# DEMANDE DE BREVET EUROPEEN

(11) **EP 1 072 217 A1**
(43) Date de publication de la demande: **31.01.2001**
(21) Numéro de dépôt: 99401943.8
(22) Date de dépôt: 29.07.1999
(51) Int. Cl.: A47D 13/08, A61F 5/37

(54) **Dispositif de maintien de posture pour personne alitée et procédé pour sa fabrication**

(71) Demandeur: Douret, Laurence, épouse Vaivre, 94250 Gentilly (FR); G D2 R, 37210 Rochecordon (FR)
(72) Inventeur: Douret, Laurence, épouse Vaivre, 94250 Gentilly (FR); Ducrocq, Gilles, 37270 Montlouis-sur-Loire (FR); Robine, Denis, 37000 Tours (FR)
(74) Mandataire: Thibon-Littaye, Annick

(57) **Abrégé**

Le dispositif de l'invention est conçu notamment pour maintenir un bébé dans la position couchée désirée. Il comprend un coussin principal longiforme (1) dans la masse de rembourrage duquel est noyée une tige d'armature (6) en un matériau à déformation réversible sélectivement par un adulte. Ce coussin présente trois tronçons successifs constituant deux éléments de soutien latéral (2 et 3) s'étendant de part et d'autre d'un pont médian pour appui fessier (4) qui les réunit et qui présente une section plus faible que celle des éléments de soutien latéral. Son procédé de fabrication consiste essentiellement à comprimer la masse de rembourrage (8) pour l'enfiler dans sa housse (9), ce pourquoi on l'enveloppe dans un film étanche (7) et, depuis l'une des extrémités, on applique une dépression qui évacue l'air remplissant les cellules de la mousse en se propageant le long de l'armature (6).

## Description

La présente invention concerne la conception et la fabrication d'un dispositif destiné à assurer un maintien de posture chez une personne alitée. On doit comprendre ici qu'en fonction des dimensions qui lui sont données, le dispositif suivant l'invention peut convenir à toutes sortes de personnes alitées, et tout aussi bien notamment à des patients adultes, malades ou non, notamment aux personnes âgées ou à tout individu atteint d'une affection plus ou moins invalidante, qu'à des enfants, plus particulièrement à des bébés, enfants en bas âge, nourrissons, nouveaux-nés à terme ou prématurés. Toutefois, comme les besoins en cette matière se situent principalement chez les bébés, on concernera plus particulièrement cette application par la suite.

Dans ses formes de réalisation préférées, celles notamment dont la conception est la plus élaborée parmi celles qui seront détaillées plus loin, le dispositif suivant l'invention présente un intérêt particulièrement important pour les milieux hospitaliers et les maternités, par le fait qu'à partir d'un même modèle commercial, il est facile de l'adapter à de multiples usages d'une personne à l'autre, de l'utiliser pour maintenir des postures variables chez un même patient, de suivre l'évolution d'un bébé au cours de sa vie en se pliant aux modifications de ses besoins d'un moment à l'autre, etc.

Suivant un état de l'art en la matière illustré par le document de brevet américain US 5 371 909, on connaît des dispositifs pour bébés qui se présentent sous la forme d'un boudin que l'on peut recourber en U ou en S en agissant sur une barre de plomb qu'il comporte. En plus des risques inhérents à l'emploi du plomb, surtout pour de jeunes enfants, ce dispositif a l'inconvénient de ne se prêter qu'à un nombre de dispositions très limitées. Il ne présente pas la souplesse d'emploi ni la sécurité qui sont exigibles dans ce genre d'application. La barre de plomb est nécessairement disposée en bordure du coussin, sur sa face inférieure, car d'une part elle doit être rendue aisément accessible pour permettre sa déformation à la main sans que l'on soit gêné par la mousse qui la surmonte, et d'autre part il est prévu qu'elle joue un rôle de lest en maintenant le boudin à plat sur sa face inférieure.

La présente invention permet d'échapper aux inconvénients des dispositifs connus à ce jour, dont en particulier ceux qui viennent d'être soulignés, et par ses particularités telles qu'elles seront précisées et décrites plus loin, elle permet de mieux répondre aux impératifs de la pratique industrielle. En particulier, elle apporte de multiples avantages du point de vue orthopédique, ainsi que pour un bon développement comportemental.

Suivant un mode de réalisation préféré, bien que non limitatif, le dispositif de l'invention est principalement constitué par un coussin longiforme comprenant deux éléments de soutien latéral, réalisés d'une seule pièce avec un pont médian qui les réunit et qui présente une section plus faible que lesdits éléments. L'ensemble est traversé par une tige d'armature longitudinale à mémoire de forme, réalisée en un matériau à déformation réversible non élastique, qui est noyée dans une masse de rembourrage en matière élastiquement compressible.

Cette matière est avantageusement une mousse synthétique, à base de polymères organiques, que l'on met en oeuvre par découpage et collage pour former, autour de l'armature, trois tronçons de boudin dont les diamètres respectifs sont déterminés en correspondance avec les éléments de soutien latéral et le pont médian.

Dans le produit fini, la masse de rembourrage est maintenue dans un état final où elle est partiellement comprimée par une housse extérieure, réalisée d'une seule pièce, dans laquelle elle est enfilée alors qu'elle est amenée dans un état de compression plus complète, ce pour quoi on l'enveloppe dans un film étanche et, depuis l'une des extrémités, on applique une dépression qui évacue l'air remplissant les cellules de la mousse en se propageant le long de l'armature. En conformité avec cette opération, on choisit avantageusement une mousse du type à haute porosité en cellules ouvertes, donc à parois éclatées, qui communiquent librement entre elles.

Par ses différentes caractéristiques, telles qu'elles ont été succinctement définies ci-avant ou qu'elles seront plus loin définies, décrites et illustrées, et telles qu'elles peuvent être avantageusement mises en oeuvre dans la pratique industrielle, séparément ou en chacune de leurs combinaisons techniquement opérantes, l'invention permet notamment de satisfaire par un modèle unique, commode à fabriquer en série pour un prix raisonnable et facile d'emploi, aux différents besoins qui se font jour, dans une maternité ou un service hospitalier de prématurés par exemple, quand on entend apporter le meilleur soin à des besoins divers de bébés différents, qu'ils soient d'ordre médical, sanitaire, physiologique, ou comportemental.

L'invention sera maintenant plus complètement décrite dans le cadre de caractéristiques préférées et de leurs avantages, en faisant référence à un mode de réalisation particulier du dispositif destiné aux bébés, notamment aux nouveaux-nés en milieu hospitalier. Cette description s'appuie sur les figures des dessins ci-inclus, dans lesquels :
- la figure 1 représente un dispositif de maintien de posture pour bébé selon l'invention, une fois mis en forme par un adulte ;
- la figure 2 représente partiellement ce dispositif suivant une vue perspective éclatée quand il est encore en forme rectiligne ;
- la figure 3 montre le dispositif en cours d'emploi pour un nouveau-né couché sur le côté, c'est-à-dire dans une position en décubitus latéral ;
- la figure 4 illustre l'utilisation du même dispositif pour un bébé couché sur le dos, c'est-à-dire en décubitus dorsal ;
- la figure 5 montre encore le même dispositif, avec un bébé maintenu en position de décubitus dorsal, mais dans une variante de réalisation où il comporte un harnais ;
- les figures 6 à 9 illustrent la mise en oeuvre du procédé appliqué pour la fabrication du dispositif de la figure 1, en représentant les éléments essentiels du dispositif au cours des principales étapes du procédé suivant l'invention.
- et la figure 10 également, mais en montrant seulement une coupe transversale de section droite à travers l'un des éléments de soutien latéral.

On commencera maintenant par décrire le en coussin longiforme 1 qui constitue la pièce principale du dispositif de l'invention. Il peut être utilisé seul ou en combinaison avec divers accessoires dont on parlera plus loin.

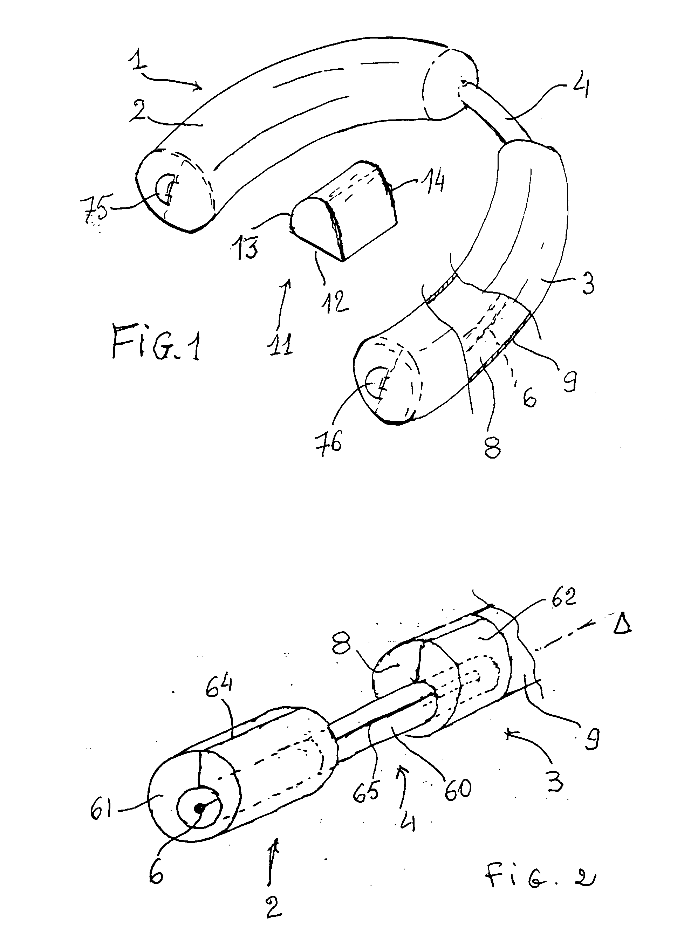

Il s'agit d'un coussin longiforme qui présente une section circulaire sur toute sa longueur dans l'exemple considéré. Dans le sens de la longueur, sa forme est rectiligne en sortie de fabrication, mais elle peut être modifiée à volonté par un adulte. Dans ses conditions normales d'utilisation, il reste conformable pour s'adapter à différentes postures, sur des bébés de tailles, poids et physiologies variés, mais la forme qu'on lui donne est toujours proche de celle qui est illustrée par la figure 1, à savoir d'une forme en U à deux branches sensiblement symétriques.

Dans l'ensemble principal que constitue le coussin 1, on distingue donc trois tronçons successifs, se situant respectivement suivant les branches du U pour les deux tronçons symétriques, et suivant la base du U pour le tronçon médian. Dans les conditions normales d'utilisation, les deux tronçons symétriques constituent pour le bébé des éléments ou boudins de soutien latéral 2 et 3, et le tronçon médian, ou pont médian 4, est apte à servir en appui fessier.

Les trois éléments sont réunis en une même pièce. Chacun présente une section circulaire de diamètre sensiblement constant sur toute sa longueur, mais ce diamètre est réduit au niveau du pont médian 4, qui présente ainsi une section plus faible que celle des éléments de soutien latéral. De plus, on peut constater sur la figure 1, et encore mieux sur la vue en perspective éclatée de la figure 2, que suivant l'axe longitudinal Δ, le pont 4 est excentré par rapport aux éléments de soutien latéral 2 et 3.

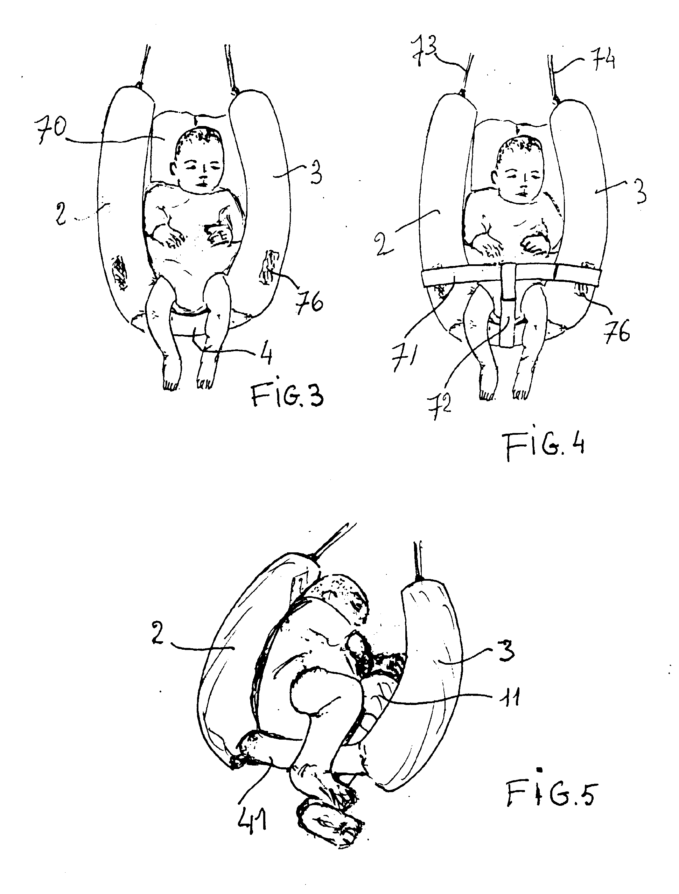

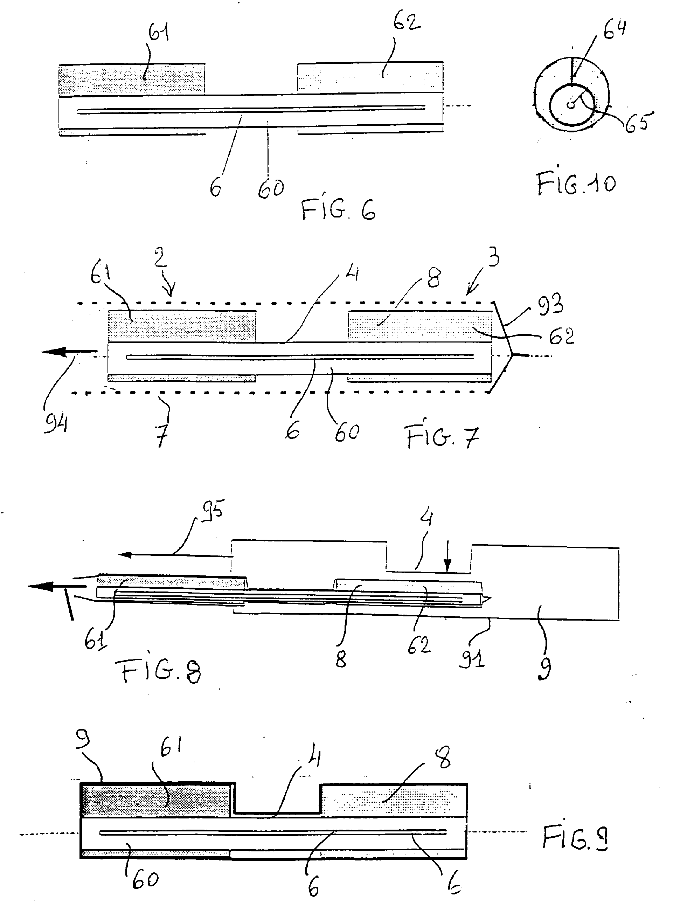

Grâce à cette disposition de l'invention, l'utilisateur peut placer le pont 4 dans une position décalée vers le dessus ou le dessous du plan, supposé horizontal, que définissent ensemble le pont 4 et les deux éléments de soutien latéral 2 et 3 quand ceux-ci sont courbés symétriquement à partir du pont 4. Cette forme sera appliquée notamment pour coucher le bébé sur le dos, comme il est illustré par les figures 3 et 4, ou pour une position en proclive sur le ventre. Pour une position en décubitus latéral comme illustré par la figure 5, on conformera plutôt le boudin de sorte que le pont 4 se place à mi-hauteur, afin que les membres inférieurs du bébé puissent s'engager à cheval dessus.

A l'intérieur, le coussin 1 est parcouru longitudinalement, sur toute la longueur des trois tronçons, par une armature, réalisée sous la forme d'une tige métallique flexible, ou tige d'armature 6, en un alliage d'aluminium à mémoire de forme. Cette armature 6 est noyée au sein d'une masse de rembourrage 8 en matériau souple, à comportement élastique en compression/ expansion, elle-même enfermée dans une housse extérieure 9.

Il est avantageux d'utiliser l'aluminium pour constituer la tige d'armature courant tout au long du coussin principal 1, en raison notamment de son prix relativement bon marché, mais aussi afin d'éviter tout risque de nocivité pour les utilisateurs, adultes, enfants, ou bébés, ayant à manipuler le coussin ou l'utilisant en maintien de posture.

La qualité de l'alliage est choisie pour que la tige d'armature 6 présente une grande flexibilité, de sorte qu'elle se laisse aisément courber à la main par une personne ayant la force normale d'un adulte et qu'elle subisse ainsi, sans rupture, des déformations importantes de type plastique, c'est-à-dire non élastique, et néanmoins réversibles. Une fois fléchie et recourbée à la forme désirée, la tige 6 peut être ramenée dans sa forme d'origine, ou dans une forme recourbée à l'inverse de celle qu'elle avait précédemment, ou encore dans toute forme désirée.

La masse de rembourrage 8 est constituée de mousse synthétique à haute porosité en cellules ouvertes, sachant que l'on désigne couramment ainsi des matières à base de résines organiques qui sont durcies par polymérisation, en présence d'un agent moussant, dans des conditions telles que l'on provoque un éclatement des parois internes.

De la sorte, les cellules de la mousse communiquent librement entre elles à travers toute la masse durcie. Naturellement, on utilise ici la notion de durcissement dans son sens courant dans l'industrie des matières plastiques, car les mousses choisies pour illustrer l'invention ne doivent pas être du type dit rigide, mais au contraire se laisser comprimer, avec réduction de volume, de manière élastiquement réversible, à partir de la forme naturelle à l'état expansé.

La masse de rembourrage contribue au fonctionnement correct de l'armature. En effet, comme elle entoure complètement la tige d'armature, elle amortit les efforts exercés, elle impose un minimum de rayon de courbure en évitant les angles trop pointus qui pourraient entraîner une fracture ou déformation permanente, elle assure un effet de bras de levier dans l'action de l'utilisateur et facilite d'autant l'obtention de la forme désirée. Par ailleurs, une fois ainsi conformé, le coussin 1 dans son ensemble conserve une certaine capacité de retour élastique en cas de déformation limitée autour de la position qui lui a été conférée.

Autour de la masse de rembourrage, l'ensemble est enveloppé dans une housse continue 9, avantageusement réalisée en un tissu doux et soyeux au toucher, même pour un bébé, imperméable et facile à nettoyer, laver et aseptiser, et souple sans être pour autant extensible. En effet, il est prévu que cette housse soit apte à jouer un rôle de contention, en maintenant la masse de rembourrage 8 comprimée entre elle et la tige d'armature interne 6 courant longitudinalement à travers les trois tronçons du coussin 1.

Dans un exemple particulier de mise en oeuvre de l'invention pris en considération ici, le diamètre de la housse 9 est tel que le volume unitaire de la masse de rembourrage se trouve réduit d'environ un tiers par rapport à celui qu'elle occupe à l'état expansé naturel. Ce diamètre est par exemple de l'ordre de 8 à 10 cm au niveau des boudins de soutien latéral 2 et 3, et seulement environ du tiers à la moitié de cette valeur au niveau du pont 4, qui présente par exemple un diamètre de 4 centimètres. Quant à la tige d'armature 6, elle présente un diamètre de l'ordre de 4 millimètres.

La figure 2 montre clairement la constitution interne du coussin longiforme constituant la pièce principale du dispositif selon l'invention. Le coussin y est supposé dans la forme qu'il présente avant d'avoir été mis en forme par l'utilisateur. On y a fait figurer la housse extérieure 9 sur une faible partie, afin de montrer plutôt la masse de rembourrage 8 qui entoure l'armature interne 6.

On y voit que cette masse de rembourrage est en réalité constitué en plusieurs pièces, chacune découpée dans un bloc de mousse synthétique. De manière avantageuse, il s'agit de trois pièces tubulaires à contour externe cylindrique, en pratique deux pièces ou tubes de plus grand diamètre auxquels on a affecté les références 61 et 62 et une pièce tubulaire ou tube central 60, de diamètre plus faible mais de longueur beaucoup plus importante.

En longueur et en diamètre externe, les dimensions des pièces tubulaires de plus grand diamètre 61 et 62 sont choisies en correspondance avec la longueur et le diamètre que présente la housse 9 au niveau des éléments de soutien latéral 2 et 3.

Le tube central, quant à lui, présente un diamètre interne égal au diamètre externe de la tige d'armature, elle-même cylindrique, ou du moins un diamètre voisin de cette dernière, en plus ou en moins, dès lors que cela ne fait pas obstacle à la mise en oeuvre de la masse de rembourrage par compression ou expansion. Son diamètre externe est choisi en correspondance avec le diamètre de la housse au niveau du pont 4, et la fraction de sa longueur qui est excédentaire par rapport à la longueur cumulée des deux tubes de grand diamètre est sensiblement égale à celle du pont 4).

On aura dès lors compris que le diamètre interne des tubes de plus grand diamètre est sensiblement égal au diamètre externe du tube central, de telle manière qu'ils se placent sans difficulté à l'intérieur de ce dernier.

Enfin, la longueur totale est quelque peu, mais sensiblement, supérieure à la longueur de la tige d'armature centrale 6. Ceci permet qu'à l'état comprimé, la mousse des pièces tubulaires formant la masse de rembourrage 8 viennent se refermer sur elles-mêmes par-dessus les bouts de l'armature et assure ainsi une protection qui rend cette dernière insensible de l'extérieur.

Toujours en référence à la figure 2, on constate que le tube central se place effectivement en position excentrée par rapport à l'axe des tubes de grand diamètre, ainsi qu'on l'a déjà indiqué. En conséquence, si le rayon du tube interne est constant tout autour de sa section droite, la tige d'armature se plaçant alors suivant son axe A, il n'en est rien pour les tubes de plus grand diamètre, dont au contraire le rayon est plus important en partie haute de la figure 2 qu'en partie basse. Cette disposition apparaît également de la vue en coupe de la figure 10.

C'est suivant leur rayon le plus long que les tubes de grand diamètre présentent une fente 64 s'étendant tout au long de la génératrice correspondante. Une fente analogue se voit en 65 pour le tube de faible diamètre 60. Ces fentes viennent de fabrication. Elles découlent naturellement du découpage de la mousse utilisée. Au cours des opérations de fabrication des boudins, elles sont collées bord sur bord une fois mises en place sur l'élément qu'elles entourent, tige 6 pour le boudin formant le tube central et le pont 4, ou tube central 60 pour les boudins latéraux.

On observe enfin des figures 2 et 10 qu'une fois l'assemblage terminé, les fentes respectives 64 et 65 sont sur des rayons différents par rapport à l'axe matérialisé par la tige 6, ce pour de simples questions de solidité.

Une fois mise en place pour envelopper la masse 8 en forme comprimée, la housse 9 n'a plus qu'à être cousue longitudinalement bord à bord sur elle-même, puis suivant un diamètre au niveau des fonds qui la termine au bout libre des boudins latéraux 2 et 3. En effet, elle est confectionnée d'une seule pièce en ce qui concerne sa paroi enveloppant le pourtour du coussin 1. Pour le comprendre, il convient de se référer au cas faisant l'objet de la figure 8, où l'on voit que, contrairement à ce qu'évoque plutôt la figure 2, il existe une arête 91 courant rectiligne d'un bout à l'autre du coussin, le long de la génératrice où l'épaisseur des tubes 61 et 62 est la plus mince.

On se référera maintenant à différents situations dans l'utilisation du dispositif dont la partie principale vient d'être décrite.

Pour un premier cas d'exemple, la figure 3 montre le coussin 1 utilisé pour le maintien de posture d'un bébé couché sur le dos, position très souvent recommandée par les pédiatres. Elle montre que le bébé est convenablement entouré de chaque côté par les boudins latéraux 2 et 3, sans être gêné dans l'exercice de mouvements verticaux. Ceci se vérifie pour ceux des bras, et aussi pour ceux des jambes, lesquelles se placent aisément par-dessus le pont 4. Mais on y voit aussi que, de part et d'autre du corps en appui sur le pont 4, il est maintenu latéralement au niveau de bassin par les boudins latéraux. Cette disposition est favorable du point de vue orthopédique. Elle évite l'apparition de la malformation courante dite culotte d'adduction.

La même figure illustre l'emploi d'un accessoire particulier du dispositif, constitué par un coussin 70, à couture médiane en diminution d'épaisseur, sur lequel repose le bébé.

Elle montre aussi que la face supérieure des boudins 2 et 3 (le pont 4 étant excentré vers leurs faces inférieures) porte une portion de ruban de fixation, du type des bandes connues sous la marque Velcro, qui se fixent sur une bande de nature complémentaire en s'accrochant lors du contact. Ces portions de bandes, évoquées sous les références 76, servent à fixer sur le coussin principal d'autres accessoires, comportant eux-mêmes au moins une portion de bande de nature complémentaire.

C'est ainsi que la figure 4 montre une bande 71, se disposant transversalement sur le bébé, de l'un à l'autre des boudins de soutien latéral. Dans ce cas, qui reste bien entendu non limitatif, cette bande appartient à un harnais, dans lequel elle est complétée par une autre bande 72, assemblée avec elle, qui se dispose dans l'entre-jambes du bébé. Pour constituer cette dernière, on utilise avantageusement du tissu qui se fixe sur lui-même et avec lequel on forme des boucles autour de la bande transversale 71, au-dessus du bébé et sous lui.

La figure 4, comme d'ailleurs la figure 3, fait d'autre part apparaître des rubans ou cordelettes, 73 et 74, que se fixent respectivement au bout libre de chacun des boudins 2 et 3, par un noeud formé en l'une de leurs extrémités autour d'un anneau, respectivement 75 et 76. Ces deux anneaux sont visibles sur la figure 1. Ils sont tenus dans la couture diamétrale qui ferme la housse 9. Les rubans ou cordelettes 73 et 74 servent alors à retenir le dispositif sur un matelas, dans un lit ou berceau ou réceptacle équivalent, en y fixant leurs extrémités libres.

Ce genre de fixation par les rubans ou cordelettes 73 et 74 est tout particulièrement utile quand le dispositif décrit est utilisé pour une posture en décubitus ventral, et en proclive, c'est-à-dire que le bébé est couché dans une position inclinée sur l'horizontale. On évite donc que le poids du bébé fasse glisser le dispositif de maintien de posture. Il est à noter que dans ce cas, le dispositif se présentera l'autre face sur le dessus, le pont 4 d'appui fessier étant dégagé pour laisser passer les jambes du bébé dessous.

La figure 5 montre une position d'utilisation du dispositif de la figure 1, dans le cas cette fois d'un bébé couché en décubitus latéral. Le coussin principal a été conformé de telle sorte que les jambes du bébé se placent aisément de manière symétrique de part et d'autre du pont d'appui fessier. Suivant le sens de courbure imposé au coussin 1 par le biais de la tige d'armature, ce pont se trouve alors en 41, dans une position qui est décalée latéralement dans le plan défini par le pont et les deux éléments de soutien latéral (plan horizontal en utilisation normale), alors que pour une posture en décubitus dorsal il se place au-dessus (figure 3) et pour une posture en décubitus ventral sur le dessous.

L'un des boudins latéraux, celui référencé 2 dans le cas représenté, sert en boudin d'appui cervico-dorsal. L'autre est nettement dégagé pour ne pas gêner le bébé.

Par contre, on utilise avantageusement un autre accessoire, constitué par coussin jouant le rôle d'une cale de maintien ventral. Cette cale 11 se fixe avantageusement de la même manière que la bande 71 sur les boudins latéraux 2 et 3 du coussin principal, éventuellement par l'intermédiaire d'un tapis dont elle est solidaire.

Elle est clairement représentée sur la figure 1, dans le cas considéré ici, où un tel tapis n'est pas prévu. En sous-face, elle comporte une portion de bande d'accrochage analogue aux portions 76 cousues sur la housse 9 sur les boudins 2 et 3. De ce fait, elle se fixe sur une bande complémentaire identique ou analogue à la bande 71 de la figure 4, qui remplit donc la même fonction qu'un tapis qui serait détachable de la cale.

Pour l'utilisation de cette cale qui en est faite suivant la figure 5, il peut être avantageux, conformément à l'invention que ce mode de fixation amovible permette de donner à la cale de maintien ventral une position réglable aussi bien dans le sens longitudinal du boudin de soutien cervico-dorsal 2 que transversalement à lui, en distance. Le même dispositif est alors commode à utiliser pour des bébés d'âges variables. Quand un même enfant grandit, on écarte plus la cale 11 du pont 4 et on la place plus loin du boudin de soutien cervico-dorsal, les deux boudins 2 ET 3 étant eux-mêmes plus écartés l'un de l'autre.

La cale 11 étant qualifiée de coussin, elle est constituée comme le coussin principal 1, d'une masse de rembourrage enveloppée sous compression dans une housse externe. Elle est par contre dépourvue d'armature interne comparable à la tige 6 décrite plus haut.

La figure 1 montre qu'elle présente un profil spécial de section droite. Sa base 12 en sous-face est plane. C'est sur elle que s'accroche la bande déjà mentionnée, constituant un ruban de liaison en fixation amovible avec le boudin de soutien latéral 2 utilisé en soutien cervico-dorsal, ou même seulement en soutien dorsal pour un bébé plus grand. En regard de ce boudin, le profil suit une courbe arrondie 13, bombée en sa direction, qui rejoint une face supérieure 14, orientée inclinée par rapport à la base 12 jusqu'à former avec elle un angle aigu 15. Au total, on s'assure ainsi d'apporter un confort optimal au bébé, tant au niveau du ventre qu'à celui de sa main libre, alors que la cale 11 est par ailleurs stabilisée au mieux dans la position qui lui conférée au départ.

On s'intéressera maintenant d'une manière plus précise, en faisant référence aux figures 6 à 9, acompagnées de la figure 10, au procédé de fabrication du coussin principal que l'on a déjà largement décrit. A cette occasion, on parlera d'un film étanche 7 (figure 7). Simplement enroulé sur plus d'un tour autour de la masse de rembourrage 8, il s'interpose en doublure interne de la housse extérieur 9.

Le procédé en question met à profit le fait que le coussin principal des dispositifs contient une tige d'armature selon son axe et que, de plus, la mousse est à cellules ouvertes, pour faciliter la circulation d'air le long de cette armature. La circulation radiale est également favorisée, mais à un degré moindre en raison de l'effet freineur de la réduction de section par la compression. En tout état de cause, la dépression par aspiration sous vide se propage aisément en longeant la tige d'armature, car il y reste toujours quelques couches de cellules qui ne se ferment pas puisque la tige 6 n'est pas compressible et que sa surface en limite du tube de mousse central ne bouge pas.

Les figures montrent les différents éléments en cours d'assemblage, dans leur évolution propre et leur évolution en relation les uns avec les autres.

A l'étape de la figure 6 (ou de la figure 10 en coupe) les trois pièces de mousse sont en place, les tubes de grand diamètre extérieur 61 et 62 entourant les tronçons latéraux du tube central 60 de petit diamètre (au niveau des boudins de soutien latéral 2 et 3), et ce dernier entourant la tige d'armature 6. Elles occupent encore un volume maximal correspondant à l'état expansé naturel de la mousse. Par collage, au moins par endroits de place en place, les fentes en 64 et 65 sont fermées et tubes et armature sont maintenues dans leurs dispositions relatives, les fentes 64 et 65 étant décalées angulairement et les tubes se terminant longitudinalement sur une même section transversale, alors que les extrémités de la tige 6 se placent en retrait aux deux bouts de l'ensemble.

Sur la figure 7, l'ensemble de la masse de rembourrage a été enveloppée d'un film souple d'étanchéité 7. On a même fermé ce film en 93, à l'une des extrémités de l'ensemble. A l'autre extrémité, la flèche 94 évoque l'aspiration sous vide. Celle-ci crée une dépression qui se propage rapidement, d'abord le long de la tige d'armature 6, et de là radialement jusqu'au pourtour extérieur des tubes 60-61-62 constituant ensemble la masse de rembourrage 8. Par voie de conséquence, le film 7 ne tarde pas à se plaquer sur la masse de rembourrage, en tous points de sa surface extérieure, ce qui suffit à assurer l'étanchéité au vide.

Le passage de la figure 7 à la figure 8 correspond à l'étape essentielle du procédé suivant la présente invention. En aspirant l'air contenu dans les cellules de la mousse du fait de la dépression appliquée depuis l'intérieur des tubes, en direction centrifuge, le volume des tubes s'est réduit très fortement sur toute la longueur de la masse 8.

La figure 8 suppose que l'on poursuit l'action de mise sous vide illustrée par la flèche 94, la masse 8 restant donc dans son état comprimé au maximum. Sous ce volume minimal, le diamètre du contour externe des tubes latéraux 61 et 62 est réduit au point d'être intérieur au diamètre du contour externe du pont 4 défini par la housse 9. On ne rencontre donc aucune difficulté à enfiler la matière de rembourrage 8 dans la housse 9 en tirant celle-ci dans le sens de la flèche 95.

Quand la mise en place de la housse 9 (préalablement fermée au moins suivant la couture longitudinale) est terminée, il ne reste plus qu'à rompre le vide pour que la mousse tende à reprendre élastiquement son volume initial. Elle en est empêchée au-delà d'un volume intermédiaire définitif imposé par le diamètre de la housse 9. On arrive alors à l'étape de la figure 9. Du côté qui était resté ouvert (à gauche de la figure), on referme complètement la mousse des tubes 60 et 61, par collage, par-dessus le bout de l'armature 6, noyée en retrait de la face extérieure de la masse de rembourrage globale, et l'on ferme la housse 9 en cette extrémité par couture.

La description qui précède explique clairement comment l'invention permet d'atteindre les objectifs qu'elle s'est fixés. On aura compris, en particulier, que le procédé suivant l'invention, impliquant d'enfiler la housse sur la masse de rembourrage temporairement comprimée sur elle-même par aspiration sous vide par l'intérieur, permet de fabriquer dans des conditions économiques satisfaisantes les coussins en forme de boudins en plusieurs tronçons de sections différentes, dont on a vu également qu'ils présentent un grand intérêt.

L'invention n'est cependant pas limitée par ce qui a été exposé pour des exemples de mise en oeuvre. Elle s'étend au contraire à de nombreuses variantes qui respectent les caractéristiques essentielles du dispositif de l'invention.

## Revendications

1. Dispositif de maintien d'une personne alitée dans une posture déterminée, caractérisé en ce qu'il comporte un ensemble ou boudin principal longiforme (1) formant coussin, qui présente trois tronçons successifs constituant deux éléments de soutien latéral (2 et 3) s'étendant de part et d'autre d'un pont médian pour appui fessier (4) qui les réunit et qui présente une section plus faible que celle des éléments de soutien latéral, ledit ensemble principal étant constitué d'une housse continue (9) enveloppant une masse de rembourrage (8) comprimée entre elle et une armature interne (6) en un matériau à mémoire de forme, capable de déformation réversible non élastique, courant longitudinalement à travers les trois tronçons.

2. Dispositif selon la revendication 1, caractérisé en ce que lesdits éléments de soutien latéral (2 et 3) sont symétriques par rapport audit pont (4) pour être utilisés alternativement chacun conformé en boudin d'appui cervico-dorsal (21) pour un patient couché en décubitus latéral, dans une position par ailleurs en appui sur ledit pont (4), et éventuellement à cheval sur ce dernier.

3. Dispositif suivant l'une quelconque des revendications 1 ou 2, caractérisé en ce que ledit pont (4) est excentré par rapport aux éléments de soutien latéral (2 et 3) suivant l'axe longitudinal (Δ), dans une position décalée vers le dessus ou le dessous d'un plan défini par ledit pont médian (4) et les deux éléments de soutien latéral (2 et 3) quand ceux-ci sont courbés symétriquement par rapport audit pont, ou dans une position (41) décalée latéralement dans ce plan, suivant le sens de courbure imposé par l'armature.

4. Dispositif suivant l'une quelconque des revendications 1 à 3, caractérisé en ce que ladite masse de rembourrage (8) est constituée de mousse synthétique à haute porosité en cellules ouvertes.

5. Dispositif suivant la revendication 4, caractérisé en ce que la masse de rembourrage (8) est réalisée en trois pièces tubulaires à contour cylindrique, comprenant un tube central (60) entourant ladite armature (6) tout au long dudit coussin, dont la section est adaptée à celle dudit pont (4), et deux tubes de plus grand diamètre (61 et 62), se disposant autour dudit tube central (60), pour remplir la housse (9) au niveau des deux tronçons constituant les éléments de soutien latéral (2 et 3).

6. Dispositif suivant la revendication 5, caractérisé en ce que chaque pièce tubulaire de la masse de rembourrage présente initialement, sur toute sa longueur, une fente radiale (64, 65), facilitant sa mise en oeuvre, qui est ensuite fermée par collage.

7. Dispositif suivant la revendication 4 ou 5, caractérisé en ce que chacune des extrémités de la masse de rembourrage (8) est collée sur elle-même en fermeture complète de la section transversale de l'élément de soutien latéral correspondant (2 ou 3), par-dessus le bout de l'armature (6), qui se trouve ainsi noyée en retrait de la face extérieure de ladite masse de rembourrage (8) en cette extrémité.

8. Dispositif suivant l'une quelconque des revendications 1 à 7, caractérisé en ce que ladite masse de rembourrage (8) est enveloppée d'un film souple d'étanchéité (7) en doublure interne à ladite housse (9).

9. Dispositif suivant l'une quelconque revendication 1 à 8, caractérisé en ce ladite housse (9) est réalisée en une seule pièce de tissu soyeux imperméable cousue longitudinalement sur elle-même.

10. Dispositif suivant l'une quelconque des revendications 1 à 9, caractérisé en ce que, pour servir notamment en posture de décubitus dorsal, il comporte en outre un coussin de tête séparé dudit ensemble principal (1) .

11. Dispositif suivant l'une quelconque des revendications 1 à 10, caractérisé en ce qu'il comporte en outre une cale de maintien ventral (11), se fixant amovible audit boudin principal (1), dans une position réglable longitudinalement et transversalement à distance de l'un desdits éléments de soutien, pour servir en combinaison avec celui-ci dans les postures de décubitus latéral où il est utilisé en appui cervico-dorsal.

12. Dispositif suivant la revendication 11, caractérisé en ce que ladite cale (11) présente une section à profil arrondi, bombé en regard de l'élément latéral coopérant, reliant une base plane d'accrochage sur un ruban de liaison avec ledit élément de soutien latéral et une face supérieure inclinée par rapport à ladite base.

13. Dispositif suivant l'une quelconque des revendications 1 à 12, caractérisé en ce qu'il comporte en outre un harnais se fixant de manière amovible sur les éléments de soutien latéral (2, 3) du boudin principal (1), ledit harnais comportant au moins une bande transversale (71) en tissu apte à se fixer par contact sur lesdits éléments de soutien latéral ou sur une cale suivant la revendication 11, et comportant avantageusement en outre une bande d'entrejambe (72).

14. Procédé de fabrication d'un coussin longiforme de dispositif de maintien de posture pour personne alitée comportant une housse continue (9) enveloppant une masse de rembourrage (8) comprimée entre elle et une armature interne (6) en un matériau à mémoire de forme, capable de déformation réversible non élastique, caractérisé en ce que qu'il consiste essentiellement à comprimer la masse de rembourrage sous un volume réduit pour l'enfiler dans ladite housse, en l'enveloppant dans un film étanche et en appliquant, depuis l'une des extrémités, une dépression qui évacue l'air remplissant les cellules de la mousse en se propageant le long de l'armature.
